Europäisches Patentamt

European Patent Office

Office européen des brevets

(11) Numéro de publication : **0 451 062 A1**

# (12) DEMANDE DE BREVET EUROPEEN

(21) Numéro de dépôt : **91420102.5**

(22) Date de dépôt : **27.03.91**

(51) Int. Cl.⁵ : **A61M 1/00**

(30) Priorité : **02.04.90 FR 9004686**

(43) Date de publication de la demande :
**09.10.91 Bulletin 91/41**

(84) Etats contractants désignés :
**AT BE CH DE DK ES FR GB GR IT LI LU NL SE**

(71) Demandeur : **Valette, Jackie**
**4 Rue Cécile Sauvage**
**F-42100 Saint Etienne (FR)**

(72) Inventeur : **Valette, Jackie**
**4 Rue Cécile Sauvage**
**F-42100 Saint Etienne (FR)**

(74) Mandataire : **Dupuis, François et al**
**Cabinet Laurent et Charras, 3 Place de**
**l'Hôtel-de-Ville, BP 203**
**F-42005 St. Etienne Cédex 1 (FR)**

(54) **Appareil pour moucher les personnes.**

(57) L'appareil est remarquable en ce que l'embout nasal est relié de manière démontable a un corps (2) constituant un réservoir de réception des mucosités du nez, ledit corps étant relié par un tuyau flexible (5) à l'enveloppe souple (6), qui présente des moyens (9) et (10) aptes à assurer le refoulement de l'air, seulement à l'extérieur, à chaque mouvement d'aspiration manuelle exercée sur ladite enveloppe.

FIG.1

EP 0 451 062 A1

L'objet de l'invention se rattache au secteur technique des appareils médicaux et d'hygiène.

Plus particulièrement, l'invention concerne les appareils pour moucher les enfants et les personnes handicapées.

On connaît des appareils présentant un embout nasal relié à une poire d'aspiration. Avec ce type d'appareil, après chaque aspiration, il est nécessaire de le retirer et d'enlever les mucosités qui apparaissent dans l'embout nasal. Compte tenu de cette conception, il n'est donc pas possible de procéder à une aspiration au moyen de la poire d'une manière continue, à défaut de renvoyer les mucosités dans la narine. En outre, pour être efficace, il est nécessaire de pratiquer une aspiration forte en lachant la poire brusquement, ce qui peut provoquer un éclatement des vaisseaux sanguins.

pour tenter de remédier à ces inconvénients, on a proposé une poire équipée d'un système de valves permettant de refouler l'air, uniquement vers l'extérieur. Cet état de la technique peut être illustré par l'enseignement du brevet US 2.890.699. Cependant, il apparait que l'embout nasal est inadapté, ce qui peut rendre l'appareil dangereux dans son utilisation. En outre, il n'y a pas de partie réellement adaptée pour récupérer les mucosités.

On connaît également des appareils comprenant un embout nasal communiquant avec un corps creux équipé pour la récupération des mucosités. Le corps est relié à un moyen bucal d'aspiration des mucosités.

Ce type d'appareils, par son principe, répond à l'objectif recherché, à savoir : enlever les mucosités contenues dans les narines en toute sécurité pour la personne à moucher. Cependant, le fait que l'on soit obligé d'aspirer par voie bucale ces mucosités n'est pas très agréable et ne garantit pas toute l'hygiène nécessaire malgré l'interposition de moyens de récupération des mucosités et éventuellement de filtration des particules pouvant être introduites dans l'appareil.

Le problème que se propose de résoudre l'invention est de pouvoir éliminer en toute sécurité les mucosités sans qu'il soit nécessaire de les aspirer par voie bucale, mais d'une manière continue manuellement au moyen d'une poire.

Un tel problème est résolu en ce que l'embout nasal est relié de manière démontable à un corps constituant un réservoir de réception des mucosités du nez, ledit corps étant relié par un tuyau flexible à l'enveloppe souple, qui présente des moyens aptes à assurer le refoulement de l'air, seulement à l'extérieur, à chaque mouvement d'aspiration manuelle exercée sur ladite enveloppe.

Pour résoudre le problème posé d'assurer, à chaque mouvement d'aspiration, le refoulement de l'air, uniquement à l'extérieur, en évitant tout risque de refoulement dans le corps, les moyens sont des clapets anti-retours dont l'un situé à l'entrée de l'enveloppe souple, ouvre le passage à l'intérieur de ladite enveloppe, lorsque celle-ci est relâchée. tandis que l'autre clapet situé de préférence à l'opposé de l'entrée, ouvre le passage vers l'extérieur lorsque l'enveloppe est pressée.

Avantageusement, ce problème est résolu en ce que les clapets sont constitués chacun d'un corps cylindrique creux rendu prisonnier d'un manchon de l'enveloppe souple et dans lequel est fixée élastiquement une bague fendue de liaison avec le tuyau flexible, la paroi arrière fermée dudit corps et l'extrémité en regard de ladite bague délimitant une chambre dans laquelle peut se déplacer lors de l'aspiration et du refoulement, un disque obturateur donnant ou interdisant le passage d'air du clapet dans l'enveloppe souple ou du clapet vers l'extérieur par un ou des orifices.

Pour résoudre le problème posé d'assurer la retenue des mucosités, le recevoir et équipé d'un tampon ou d'un bouchon filtre.

Dans une autre forme de réalisation, l'embout nasal est équipé au niveau de sa partie de raccordement avec le corps de l'appareil, directement ou d'une manière rapportée, d'au moins un moyen apte à assurer la retenue des mucosités et présentant un orifice réduit pour mettre en communication l'embout nasal avec l'enveloppe souple.

Un autre problème que se propose de résoudre l'invention, dans une autre forme de réalisation de l'appareil, est de pouvoir jeter l'embout nasal après chaque utilisation.

Un tel problème est résolu en ce que l'embout nasal présente des moyens de retenue faisant office de butée à un organe apte à assurer la retenue des mucosités, tout en permettant l'aspiration.

L'organe est constitué par un disque, en communication avec un conduit coaxial dirigé à l'intérieur du corps.

Avantageusement, suivant une autre caractéristique, l'embout nasal est réalisé en matière transparente afin de visualiser les mucosités.

Ces caractéristiques et d'autres encore ressortiront de la description qui suit.

Pour fixer l'objet de l'invention sans toutefois le limiter, dans les dessins annexés:

La figure 1 est une vue en coupe partielle illustrant un appareil selon une première forme de réalisation comprenant un embout nasal, un corps-réservoir, un flexible et un organe manuel d'aspiration des mucosités.

Les figures 2 et 3 sont des vues en coupe à plus grande échelle montrant l'organe manuel respectivement en position de refoulement et d'aspiration d'air.

La figure 4 est une vue en coupe, à plus grande échelle encore, d'un des clapets équipant l'organe manuel.

La figure 5 est une vue illustrant une variante de réalisation du corps-réservoir et de l'embout nasal.

La figure 6 est une vue en coupe illustrant un autre exemple de réalisation de l'appareil selon l'invention.

La figure 7 est une vue en coupe, d'une autre forme de réalisation de l'appareil, notamment au niveau de son embout.

Afin de rendre plus concret l'objet de l'invention, on le décrit maintenant d'une manière non limitative en se référant aux exemples de réalisation des figures des dessins.

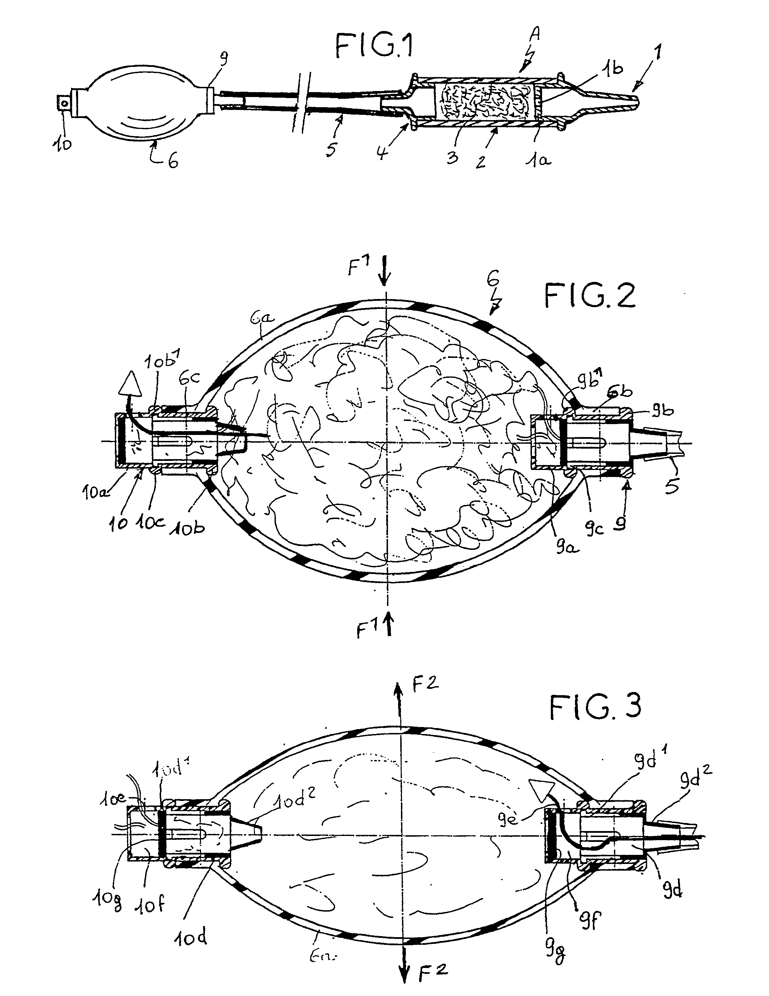

Selon les figures 1 à 4, l'appareil (A) comprend successivement un embout bucal (1) relié de manière démontable à l'extrémité d'un corps tubulaire (2) formant réservoir dans lequel on place un produit de récupération des mucosités du type tampon de coton (3) ou autre. L'autre extrémité du corps présente de manière démontable ou non un embout (4) relié par l'intermédiaire d'un tuyau flexible (5) à un organe manuel (6) sous forme d'une poir notament pour l'aspiration et le refoulement de l'air.

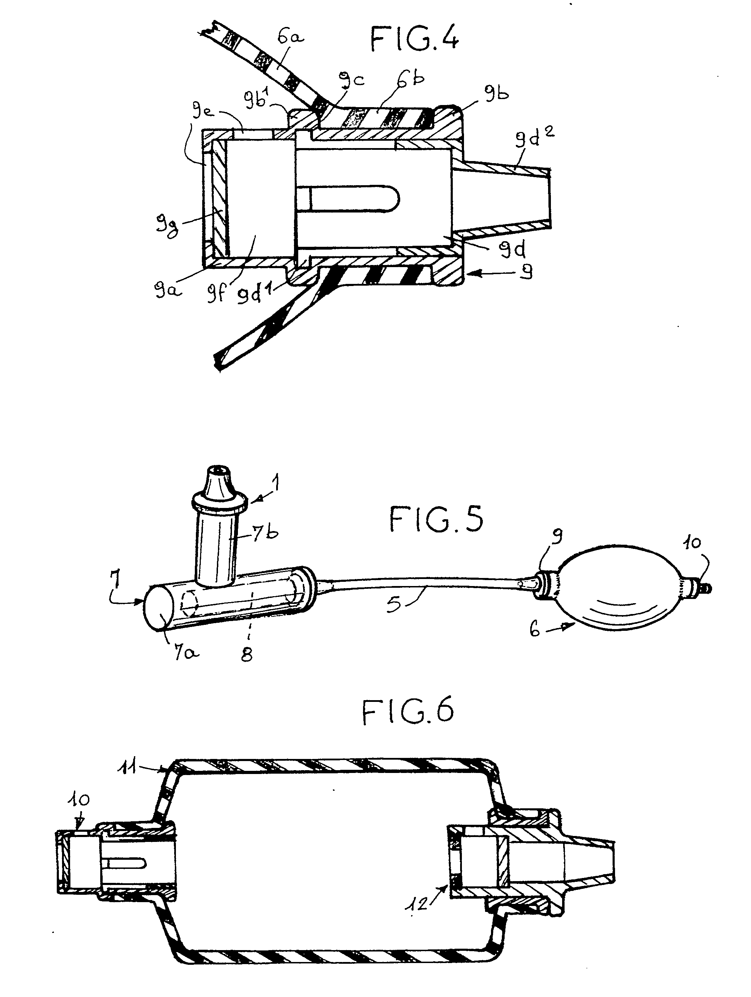

Dans la forme de réalisation illustrée à la figure 5, le corps-réservoir (7) est constitué de deux tubes (7a - 7b) formant sensiblement un T. Le tube (7a) est fermé une extrémité et reçoit à son autre extrémité un bouchon-filtre (8) relié par le flexible (5) l'organe manuel (6), tandis que le tube (7b) porte à son extrémité libre l'embout nasal démontable (1).

L'organe (6) présente en deux points, de préférence opposés, des manchons cylindriques (6b) (6c) destinés à la fixation des moyens d'aspiration et de refoulement selon l'invention.

Ces moyens sont des clapets (9) (10) de préférence identiques pour limiter les coûts de fabrication. Le clapet (9) monté sur le manchon (6b) est raccordé au flexible (5) et appelé clapet d'aspiration, tandis que le clapet (10), monté sur le manchon (6c) est appelé clapet de refoulement.

Chaque clapet est constitué par un corps cylindrique creux (9a - 10a) positionné dans les manchons (6b - 6c) entre des épaulements (9b - 10b). L'alésage du corps de clapet présente au niveau de l'épaulement médian (9b1 - 10b1) une gorge (9c - 10c) dans laquelle s'engage la collerette d'extrémité (9d1 - 10d1) d'une bague fendue (9d - 10d) dont l'autre extrémité conique constitue un embout (9d2 - 10d2) de raccordement avec le flexible (5) pour ce qui concerne le clapet d'aspiration (9).

Le corps cylindrique creux (9a - 10a) est fermé à l'opposé de l'embout (9d2 - 10d2) et présente un ou plusieurs orifices de passage d'air (9e - 10e). Comme on le voit aux figures 2 et 3, pour le clapet d'aspiration (9), les orifices (9e) communiquent avec le volume interne de l'enveloppe (6a), tandis que pour le clapet de refoulement (10), les orifices (10e) communiquent avec l'extérieur.

Enfin, dans la chambre (9f - 10f) délimitée par la paroi de fond du corps cylindrique (9a - 10a) et par la collerette (9d1 - 10d1) de la bague fendue (9d - 10d), est monté un disque (9g - 10g) ou obturateur proprement dit.

Le fonctionnement de l'appareil ainsi réalisé est le suivant : Après avoir introduit l'embout nasal (1) dans la narine à nettoyer, on presse manuellement (flèches F1), la poire (6), ce qui a pour effet de plaquer le disque obturateur (9g) du clapet d'aspiration (9) contre la bague (9d) en fermant ainsi le passage vers le flexible (5) ; l'air contenu dans la poire repousse alors le disque obturateur (10g) du clapet de refoulement (10) vers la paroi de fond du corps en permettant ainsi le passage de l'air par l'orifice (10e) vers l'extérieur (figure 2). Lorsqu'on relâche la poire (flèches F2). Le disque obturateur (10g) est attiré contre la bague (10d), tandis que l'autre disque obturateur (9g) est plaqué sur la paroi de fond du corps du clapet (9) en créant ainsi un effet d'aspiration dans le flexible (5), le corps (2) ou (7) et l'embout nasal (1) permettant l'attraction des mucosités et leur récupération par le bouchon-filtre (8) ou le tampon (3) (figure 3).

Selon une variante de réalisation illustrée à la figure 6, l'organe annuel d'aspiration et de refoulement est relié directement à un embout nasal. Dans ce cas, ledit organe à enveloppe souple (11) constitue lui-même le réservoir de récupération des mucosités.

Dans cette réalisation, on retrouve le clapet de refoulemnt (10), tandis que le clapet d'aspiration est de préférence monobloc avec l'embout nasal, l'ensemble (12) étant démontable pour permettre le vidage et le nettoyage de l'enveloppe-réservoir.

Dans une variante de réalisation, l'embout nasal (1) est équipé au niveau de sa partie de raccordement avec le corps de l'appareil, directement ou d'une manière rapportée, d'au moins un moyen (1a) apte à assurer la retenue des mucosités. Par exemple, ce moyen (1a) peut être constitué par une rondelle présentant un orifice réduit (1b) pour mettre en communication l'embout nasal avec le moyen d'aspiration.

L'embout nasal est réalisé en matière transparente afin de visualiser les mucosités.

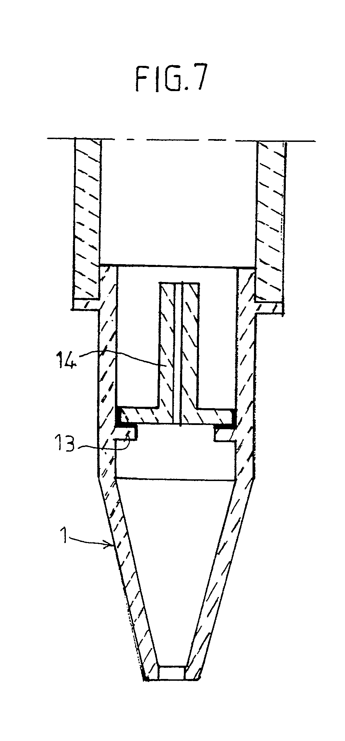

On prévoit également une autre forme de réalisation de l'appareil, selon laquelle, l'embout nasal (1) est conformé pour être jeté après chaque utilisation. Dans ce but, et comme le montre la figure 7, l'embout nasal (1) présente des moyens de retenue (13) faisant office de butée à un organe (14) apte à assurer la retenue des mucosités, tout en permettant l'aspiration. Les moyens de retenue (13) constituent une collerette et sont formés directement, lors de l'injection de l'embout nasal (1).

L'organe (14) est constitué par un disque (14a), en communication avec un conduit coaxial (14b), dirigé à l'intérieur du corps (2), après mise en place dudit organe. Ce conduit coaxial (14b) présente un orifice de diamètre déterminé, pour permettre comme indiqué, la retenue des mucosités, sans pour autant, supprimer l'effet d'aspiration.

Avantageusement, cet organe (14), qui fait office de filtre, est rapporté sur la collerette (13, en y étant fixé notamment par collage.

Les avantages ressortent bien de la description. On souligne notamment l'aspiration efficace et en toute sécurité des mucosités sans contact avec la bouche, rendant plus agréable et très hygiénique l'utilisation de l'appareil.

## Revendications

**-1-** Appareil pour moucher les personnes comprenant un embout nasal et une enveloppe souple déformable apte à créer un effet d'aspiration, caractérisé en ce que l'embout nasal est relié de manière démontable à un corps (2) constituant un réservoir de réception des mucosités du nez, ledit corps étant relié par un tuyau flexible (5) à l'enveloppe souple (6), qui présente des moyens (9) et (10) aptes à assurer le refoulement de l'air, seulement à l'extérieur, à chaque mouvement d'aspiration manuelle exercée sur ladite enveloppe.

**-2-** Appareil selon la revendication 1, caractérisé en ce que les moyens autorisant le refoulement de l'air seulement à l'extérieur après chaque aspiration annuelle sont des clapets anti-retours dont l'un (9) situé à l'entrée de l'enveloppe souple (6), ouvre le passage à l'intérieur de ladite enveloppe, lorsque celle-ci est relâchée, tandis que l'autre clapet (10) situé de préférence à l'opposé de l'entrée, ouvre le passage vers l'extérieur lorsque l'enveloppe est pressée.

**-3-** Appareil selon la revendication 2, caractérisé en ce que les clapets (9 - 10) sont constitués chacun d'un corps cylindrique creux (9a - 10a) rendu prisonnier d'un manchon de l'enveloppe souple (6) et dans lequel est fixée élastiquement une bague fendue (9d - 10d) de liaison avec le tuyau flexible (5 ), la paroi arrière fermée dudit corps et l'extrémité en regard de ladite bague délimitant une chambre (9f - 10f) dans laquelle peut se déplacer lors de l'aspiration et du refoulement, un disque obturateur (9g - 10g) donnant ou interdisant le passage d'air du clapet (9) dans l'enveloppe souple (6 ) ou du clapet ( 10 ) vers l'extérieur par un ou des orifices (9e - 10e).

**-4-** Appareil selon la revendication 1, caractérisé en ce que le réservoir (2) et équipé d'un tampon (3) ou d'un bouchon filtre (8).

**-5-** Appareil selon la revendication 1, caractérisé en ce que l'embout nasal (1) est équipé au niveau de sa partie de raccordement avec le corps de l'appareil, directement ou d'une manière rapportée, d'au moins un moyen (1a) apte à assurer la retenue des mucosités et présentant un orifice réduit pour mettre en communication l'embout nasal avec l'enveloppe souple (6).

**-6-** Appareil selon la revendication 1, caractérisé en ce que l'embout nasal (1) présente des moyens de retenue (13) faisant office de butée à un organe (14) apte à assurer la retenue des mucosités, tout en permettant l'aspiration.

**-7-** Appareil selon la revendication 6, caractérisé en ce que l'organe (14) est constitué par un disque, en communication avec un conduit coaxial dirigé à l'intérieur du corps (2).

**-8-** Appareil selon la revendication 6, caractérisé en ce que l'organe (14) est fixé sur les moyens (13).

FIG.1

FIG.2

FIG.3

FIG.4

FIG.5

FIG.6

# FIG.7

14

13

1

**Office européen des brevets**

# RAPPORT DE RECHERCHE EUROPEENNE

Numero de la demande

EP 91 42 0102

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,P | US-A-4 921 488 (C. MAITZ et al.) * Figures; colonne 1, lignes 6-10; colonne 3, ligne 50 - colonne 4, ligne 12; colonne 4, lignes 57-62 * | 1,2 | A 61 M 1/00 |
| Y | --- | 5 | |
| A | FR-A-2 473 317 (J. DANON) * Figure 3; page 4, lignes 3-18 * --- | 1 | |
| Y | US-A-2 890 699 (L. MILLER) * Figures; colonne 1, lignes 37-50; colonne 2, lignes 10-58 * --- | 5 | |
| A | US-A-4 643 719 (G. GARTH et al.) * Figures 3,5,6,8,10; colonne 4, lignes 34-45; colonne 5, ligne 60 - colonne 6, ligne 3 * --- | 1-3 | |
| A | CA-A-1 136 018 (G. BERNIER) --- | 4 | |
| A | DE-A-1 917 794 (H. OBELDOBEL) ----- | | |

**DOMAINES TECHNIQUES RECHERCHES (Int. Cl.5)**

A 61 M

Le présent rapport a été établi pour toutes les revendications

| Lieu de la recherche | Date d'achèvement de la recherche | Examinateur |
|---|---|---|
| LA HAYE | 06-06-1991 | VEREECKE A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

........................................................................
& : membre de la même famille, document correspondant